# EUROPÄISCHE PATENTSCHRIFT

(11) **EP 1 066 801 B1**
(45) Veröffentlichungstag und Bekanntmachung des Hinweises auf die Patenterteilung: **22.09.2004**
(21) Anmeldenummer: 00114401.3
(22) Anmeldetag: 05.07.2000
(51) Int. Cl.: A61C 13/00, A61F 2/30

(54) **Verfahren zur Erstellung von medizinischen, insbesondere zahnmedizinischen Passkörpern**
Method and device for the manufacture of medical in particular dental prosthesis
Procédé et dispositif pour fabriquer des prothèses médicales en particulier dentaires

(30) Priorität: 05.07.1999 DE 19930859
(43) Veröffentlichungstag der Anmeldung: 10.01.2001
(73) Patentinhaber: Sirona Dental Systems GmbH, 64625 Bensheim (DE)
(72) Erfinder: Rothenberger, Bernd, 76593 Gernsbach (DE); Wedler, Volker, 68542 Heddesheim (DE); Basler, Franz, 69514 Laudenbach (DE)
(74) Vertreter: Sommer, Peter

(56) Entgegenhaltungen:
- EP-A- 0 455 853
- DE-A- 4 030 175
- US-A- 4 707 793
- US-A- 4 766 704
- US-A- 5 083 280
- US-A- 5 128 870

## Beschreibung

Die Erfindung betrifft ein Verfahren zur Erstellung von medizinischen, insbesondere zahnmedizinischen Paßkörpern, bei dem ein Werkstück, aus dem der Paßkörper erstellt wird, mittels eines Werkzeuges abtragend bearbeitet wird.

Ein solches Verfahren ist beispielsweise aus der EP-A-0 182 098 bekannt. Ein Werkstück aus dentalkeramischem Werkstoff ist auf einem Halter angeordnet, der sich bei der Bearbeitung drehend vorschiebt. Hierbei wird das Werkstück wahlweise von einer Trennscheibe oder einem fingerförmigen Schleifstift bearbeitet, die an einem Werkzeugträger angebracht sind. Die Höhenlage des Werkzeugträgers wird dabei nach Programm so gesteuert, daß die Formgebung durch den Materialabtrag entlang der zu bildenden Kontur des Paßkörpers erfolgt. Die zu bildende Kontur wurde zuvor aus einer optischen Abtastung des vorbehandelten Zahnes gewonnen, in den der zahnmedizinische Paßkörper eingesetzt werden soll.

Auch aus der EP-A-0 455 853 ist ein Verfahren zur Erstellung von zahnmedizinischen Prothetik-Paßkörpern bekannt. Hier erfolgt die Bearbeitung simultan durch zwei an unterschiedlichen Werkzeugköpfen gelagerte Werkzeuge.

Bei der Bearbeitung des Werkstücks tritt das Problem auf, daß die Abtragsleistung der Werkzeuge entlang der zu bildenden Kontur des Paßkörpers meist nicht konstant ist.

An manchen Stellen der Kontur erfolgt ein sehr starker Materialabtrag, insbesondere wenn ein Paßkörper mit steilen Flanken oder starken Vertiefungen ausgearbeitet werden soll. An anderen Stellen dagegen kann der Materialabtrag sehr gering sein, z.B. wenn eine flache, glatte Oberfläche erzeugt werden soll.

Eine zu hohe Abtragsleistung kann zu einer Überhitzung des Werkzeugs bis hin zu seiner Zerstörung führen. Es ist also in hohem Maße wichtig, dafür zu sorgen, daß während der gesamten Bearbeitung des Werkstücks eine maximale Belastung der Werkzeuge nicht überschritten wird.

Zu diesem Zwecke wurde vorgeschlagen, bei konstanter Rotationsfrequenz des rotierenden Werkzeugs laufend die Leistungsaufnahme des Rotationsantriebs zu überwachen. Diese Leistungsaufnahme stellt dann ein Maß für die momentane Belastung des Werkzeugs dar. Überschreitet die Leistungsaufnahme einen bestimmten Maximalwert, so wird die Vorschubgeschwindigkeit zwischen Werkstück und Werkzeug soweit reduziert, daß die Leistungsaufnahme wieder unter den Maximalwert sinkt.

Mit einem solchen Verfahren kann zwar in vielen Fällen eine zufriedenstellende Regelung erreicht werden. Dennoch reicht diese Regelung oftmals nicht aus, um Leistungsspitzen, die zu stark erhöhtem Verschleiß des Werkzeugs führen können, zuverlässig zu verhindern. Solche Leistungsspitzen können insbesondere dann auftreten, wenn schnelle Wechsel zwischen einer niedrigen Abtragsleistung und einer hohen Abtragsleistung stattfinden. Dies ist zum Beispiel häufig bei der Herausarbeitung von scharfkantigen Strukturen mit steilen Flanken der Fall, wie sie häufig bei Zahnkronen auftreten.

Es stellt sich also die Aufgabe, ein Verfahren zur Erstellung von medizinischen Paßkörpern anzugeben, bei dem Belastungsspitzen der Werkzeuge wirksam vermieden werden und bei dem der Paßkörper in möglichst kurzer Zeit gefertigt werden kann.

Diese Aufgabe wird gelöst durch ein Verfahren zur Erstellung von medizinischen, insbesondere zahnmedizinischen Paßkörpern, gemäß Anspruch 1.

Indem die zu erwartende Abtragsleistung des Werkzeuges schon im voraus berechnet wird, können selbst sehr schnelle Lastwechsel vorzeitig erkannt werden und bei der Steuerung der Bewegung zwischen Werkstück und Werkzeug berücksichtigt werden. Auf diese Weise werden Leistungsspitzen von vornherein wirksam ausgeschlossen. Während zum Beispiel für diejenigen Teile der Bahn, bei denen eine hohe Abtragsleistung zu erwarten ist, von vornherein eine niedrige Geschwindigkeit zwischen Werkzeug und Werkstück gewählt wird, kann die Bewegung in Regionen mit nur geringem Abtrag sehr schnell erfolgen. Dies führt zu einer erheblichen Zeitersparnis bei der Herstellung der Paßkörper. Ferner werden die Kräfte zwischen Werkzeug und Werkstück reduziert.

Dieses Verfahren läßt sich insbesondere dann sinnvoll einsetzen, wenn das Werkzeug im wesentlichen zylindersymmetrisch ist und mittels eines Rotationsantriebs um seine Symmetrieachse rotiert. Vorteilhaft ist das Werkzeug ein im wesentlichen zylindrischer Schleifstift mit einer Mantelfläche und einer Stirnfläche.

Es bieten sich insbesondere zwei Möglichkeiten für die Ermittlung der momentanen Belastung des Werkzeugs an. Gemäß der ersten Möglichkeit wird als Maß für die tatsächliche Belastung des Werkzeuges die Rotationsfrequenz des Werkzeuges bei im wesentlicher konstanter Leistungsaufnahme des Rotationsantriebs ermittelt. Gemäß der zweiten Möglichkeit wird als Maß für die tatsächliche Belastung die Leistungsaufnahme des Rotationsantriebs bei vorgegebener Rotationsfrequenz ermittelt.

Die Arten der Relativbewegung zwischen dem Werkzeug und dem Werkstück sind häufig durch die Vorrichtung, mit der der Paßkörper erstellt wird, vorgegeben. Das erfindungsgemäße Verfahren läßt sich insbesondere dann gut einsetzen, wenn die Relativbewegung zwischen dem Werkzeug und dem Werkstück folgende Bewegungsarten umfaßt:
a) linerarer Vorschub zwischen dem Werkzeug und dem Werkstück entlang einer Vorschubrichtung, die im wesentlichen senkrecht zur Symmetrieachse des Werkzeuges ist;
b) Tiefenänderung des Werkzeuges im Werkstück im wesentlichen entlang der Symmetrieachse des Werkzeuges; und
c) eindimensionale Seitwärtsbewegung zwischen dem Werkzeug und dem Werkstück entlang eines vorgegebenen Weges in einer Ebene, die im wesentlichen senkrecht zur Symmetrieachse des Werkzeuges verläuft und verschieden von der Richtung des linearen Vorschubs ist.

Die eindimensionale Seitwärtsbewegung besteht vorzugsweise in einer Verschwenkung des Werkzeuges um eine Achse, die im wesentlichen parallel zur Symmetrieachse des Werkzeuges ist. Denkbar ist aber auch ein transversales Verschieben des Werkzeuges entlang einer Richtung, die im wesentlichen senkrecht sowohl zur Symmetrieachse des Werkzeuges als auch zur Richtung des linearen Vorschubs ist.

Um komplizierte Strukturen aus dem Werkstück herausarbeiten zu können, kann es sinnvoll sein, wenn die Relativbewegung zwischen dem Werkzeug und dem Werkstück weiterhin eine Drehung des Werkstückes um eine Achse umfaßt, die im wesentlichen parallel zur Richtung des linearen Vorschubs ist.

Vorteilhaft wird die gewünschte Kontur aus dem Werkstück in einer solchen Weise herausgearbeitet, daß der lineare Vorschub schrittweise der gesamten Bearbeitung in der selben Richtung erfolgt. Der Vorschub erfolgt dabei sinnvollerweise in Schritten, die klein gegenüber dem mittleren Durchmesser des Werkzeuges sind. Die Bahn wird in Abschnitte unterteilt, zwischen denen kein linearer Vorschub erfolgt. Die zu erwartende Belastung wird dann für jeden Abschnitt aus dem Vergleich der Bahn entlang dieses Abschnittes und der Bahn des jeweils vorhergehenden Abschnitts berechnet. Jeder Bewegungsabschnitt läßt sich aus den genannten Bewegungsarten b) und c), daß heißt aus einer Tiefenänderung des Werkzeuges und einer eindimensionalen Seitwärtsbewegung, zusammensetzen. Die Berechnung der zu erwartenden Abtragsleistung kann dabei punktweise an mindestens einem vorgewählten Punkt des Bewegungsabschnitts erfolgen.

Handelt es sich bei dem Werkzeug um einen zylindrischen Schleifstift, ist es vorteilhaft, die Berechnung der zu erwartenden Belastung auf die folgende Weise vorzunehmen: Zur Bestimmung eines Maßes für die zu erwartende Belastung bei einer Tiefenänderung des Schleifstiftes im Werkstück wird näherungsweise derjenige Teil der Stirnfläche bestimmt, an dem ein Materialabtrag erfolgt. Die Geschwindigkeit der Tiefenänderung des Werkzeuges im Werkstück wird dann auf eine Maximalgeschwindigkeit begrenzt, die unter Zugriff auf eine Tabelle ermittelt wird, in der für mehrere Werte des Anteiles der Stirnfläche, an der ein Materialabtrag erfolgt, die zugeordnete Maximalgeschwindigkeit gespeichert ist.

Auf ähnliche Weise erfolgt die Regelung der eindimensionalen Seitwärtsbewegung. Zur Bestimmung eines Maßes für die zu erwartende Belastung bei der eindimensionalen Seitwärtsbewegung wird die Höhe desjenigen Teiles der Mantelfläche des Schleifstiftes berechnet, an dem ein Materialabtrag erfolgt. Die Geschwindigkeit der eindimensionalen Seitwärtsbewegung wird dann auf eine Maximalgeschwindigkeit begrenzt, die unter Zugriff auf eine Tabelle ermittelt wird, in der für mehrere Werte der Höhe desjenigen Anteil der Mantelfläche, an dem ein Materialabtrag erfolgt, die zugeordnete Maximalgeschwindigkeit gespeichert ist.

Vorteilhafte Ausgestaltungen des erfindungsgemäßen Verfahrens werden anhand der Zeichnung erläutert. Es zeigen:
- Fig. 1: Eine Prinzipskizze zur Bearbeitung des Werkstücks,
- Fig. 2: die Lage des Schleifstiftes im Werkstück in einer ersten Bearbeitungsphase,
- Fig. 3: die Lage des Schleifstiftes im Werkstück in einer zweiten Bearbeitungsphase, sowie
- Fig. 4: einen schematischen Plan zum Ablauf der Regelung.

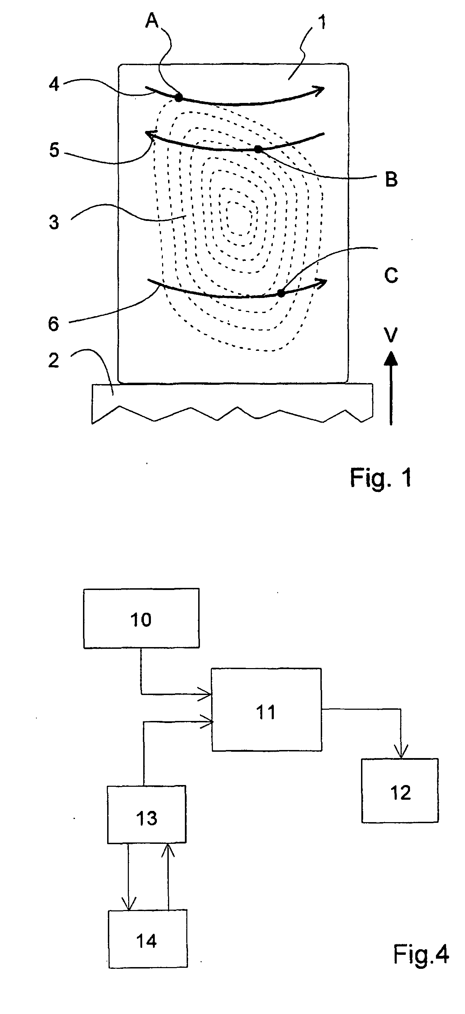

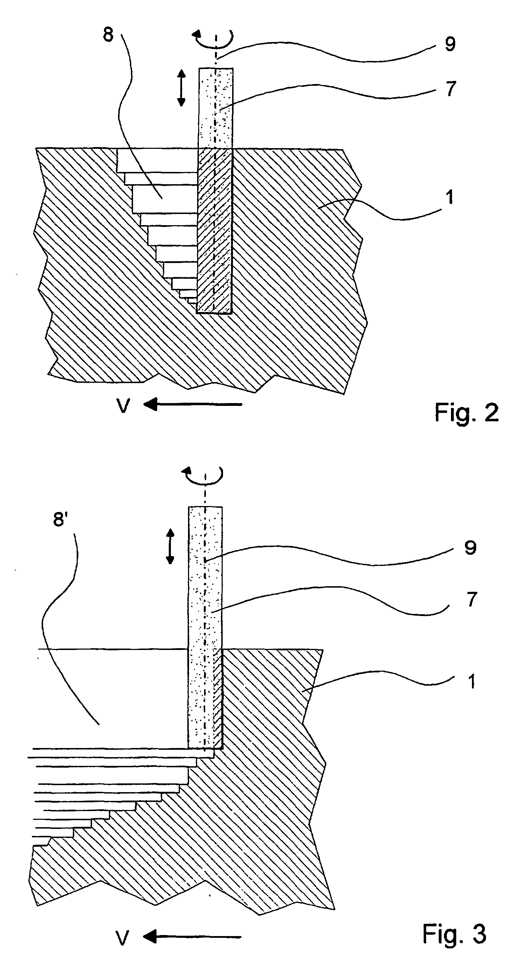

In der Fig. 1 ist ein Werkstück 1 auf einem Halter 2 montiert. Dieser ist in einer nicht dargestellten Werkstückspindel eingespannt und mittels der Werkstückspindel in axialer Richtung entlang der Vorschubrichtung V verschiebbar. Oberhalb der Bildebene ist ein nicht dargestellter Zylinderschleifer angeordnet, welcher in einer Werkzeugspindel gelagert und mittels eines Rotationsantriebs in Rotation versetzbar ist. Die Rotation des Zylinderschleifers erfolgt um eine Achse, die senkrecht auf der Bildebene und damit auch senkrecht auf der Vorschubrichtung V steht.

Mittels einer geeigneten Antriebsvorrichtung kann der Zylinderschleifer in der Werkzeugspindel entlang der Rotationsachse des Werkzeuges, also senkrecht zur Bildebene, auf das Werkzeug zu und von diesem wegbewegt werden. Dies entspricht einer Tiefenänderung des Zylinderschleifers im Werkstück entlang der Symmetrieachse des Schleifers. Zusätzlich kann die Werkzeugspindel um eine nicht dargestellte Achse in der Bildebene verschwenkt werden. Hierbei beschreibt der Zylinderschleifer eine kreisbogenförmige Bahn relativ zum Werkstück. Einige solche Bahnen, abhängig vom momentanen Betrag des Vorschubs des Werkstücks entlang V, sind in der Fig. 1 mit 4, 5 und 6 bezeichnet.

Zur Illustration des Bewegungsablaufes bei der Bearbeitung des Werkstücks sei angenommen, daß im Zuge der Bearbeitung eine Kontur im Werkstück 1 erzeugt werden soll, wie sie durch die Konturlinien 3 angedeutet ist. Diese Kontur besteht demnach in einer unregelmäßig geformten Vertiefung im Werkstück 1 in die Bildebene hinein. Eine solche Kontur kann zum Beispiel die Kaufläche einer Zahnkrone sein, die aus dem Werkstück gefertigt werden soll.

Zu Anfang der Bearbeitung befindet sich die Werkstückspindel mit dem Halter 2 und dem daran befestigten Werkstück 1 in ihrer Ausgangsstellung bezüglich der Vorschubrichtung V. Der Zylinderschleifer befindet sich dadurch bezüglich der Vorschubrichtung V am oberen Ende des Werkstücks 1. Nach einem Null-Abgleich der Werkzeuge, wie er beispielsweise in der EP-A-0 455 853 beschrieben ist, beginnt der eigentliche Bearbeitungsvorgang. Hierzu wird der Schleifstift durch einen geeigneten Vorschub des Werkstücks in Richtung V und ein geeignetes Verschwenken der Werkzeugspindel an die Position A gebracht. Der rotierende Schleifstift beginnt nun mit der Bearbeitung des Werkstücks, indem er entlang seiner Rotationsachse auf das Werkstück zubewegt wird, bis seine Stirnfläche das Werkstück berührt und mit dem Materialabtrag beginnt.

Sobald der Schleifstift um den gewünschten Betrag in das Werkstück eingedrungen ist, wird die Werkzeugspindel entlang der Richtung 4 verschwenkt, wobei kontinuierlich oder schrittweise jeweils eine Tiefenänderung des Schleifstiftes im Werkstück erfolgt. Auf diese Weise wird der erste Bahnabschnitt der herauszuarbeitenden Kontur erzeugt.

Sobald die Bearbeitung entlang dieses Abschnitts abgeschlossen ist, wird das Werkstück 1 um einen kleinen Betrag entlang der Vorschubrichtung V verschoben. Die Größe des Vorschubschnittes hängt dabei insbesondere von der gewünschten Präzision bei der Herausarbeitung der Kontur ab. Er ist jedoch in jedem Falle sehr viel kleiner als der Durchmesser des Schleifstiftes.

Nach Ausführung dieses Vorschubschrittes wird nun die Werkzeugspindel entlang einer parallel zur Richtung 4 verlaufenden Bahn zurückverschwenkt, wobei kontinuierlich die Tiefe des Schleifstiftes im Werkstück verändert wird und so ein zweiter Abschnitt der zu erzeugenden Kontur herausgearbeitet wird. Diese Abfolge aus Vorschubschritten und anschließendem Herausarbeiten der Kontur entlang eines Bahnabschnitts durch Verschwenken der Werkzeugspindel und Tiefenänderung des Werkzeuges wird nun solange fortgesetzt, bis die gesamte Kontur herausgearbeitet ist. Dabei erfolgt der Vorschub immer in der selben Richtung entlang der Vorschubrichtung V.

Die Figuren 2 und 3 illustrieren das Abtragen des Werkstückmaterials in verschiedenen Phasen während der Bearbeitung des Werkstückes 1. Die Darstellung der Figuren 2 und 3 erfolgt im Schnitt von der Seite, wobei die Blickrichtung bezüglich der Fig. 1 von links innerhalb der Bildebene liegt. Die Situation der Fig. 2 entspricht dabei etwa dem Zustand am Punkt B, die Situation der Fig. 3 in etwa dem Zustand am Punkt C. Sowohl die Fig. 2 als auch die Fig. 3 sind dabei als stark schematisierende Darstellung zu verstehen, sind also insbesondere nicht maßstabsgetreu.

In der Fig. 2 hat der Schleifstift 7 nach mehreren Vorschubschritten die dargestellten Position im Werkstück 1 erreicht. Hierbei hat er den Bereich 8 der gewünschten Kontur aus dem Werkstück 1 herausgearbeitet. Nach einem weiteren Vorschubschritt des Werkstücks 1 in Richtung V arbeitet der Schleifstift 7 nun einen weiteren Abschnitt der Kontur aus dem Werkstück 1 heraus. Hierzu wird er mit der Werkzeugspindel verschwenkt, was einer Bewegung in die Bildebene hinein oder aus ihr heraus entspricht, und seine Tiefe im Werkstück wird verändert, was einer Bewegung des Schleifstiftes nach oben oder unten entlang der Rotationsachse 9 entspricht.

Dasselbe gilt in analoger Weise für die Fig. 3. Es ist nun ein größerer Teil 8' der gewünschten Kontur im Werkstück 1 herausgearbeitet. Auch hier erfolgt nach jedem Vorschubschritt in Richtung V die Bewegung des Werkzeuges in die Papierebene hinein oder aus ihr heraus bzw. nach oben oder unten entlang der Rotationsachse 9.

Während der Bearbeitung des Werkstückes 1 lassen sich verschiedene Belastungsarten oder Lastfälle für den Schleifstift 7 unterscheiden. Der erste Lastfall ist der sogenannte stirnseitige Lastfall. Dieser tritt auf, wenn der Schleifstift 7 entlang seiner Rotationsachse in das Werkstück 1 hinein verfahren wird. Ein Materialabtrag erfolgt hier nur an der Stirnseite des Schleifstiftes. Hierbei sind verschiedene Situationen zu unterscheiden. In der ersten Situation nimmt die gesamte Stirnfläche des Schleifstiftes am Materialabtrag teil. Dies ist zum Beispiel dann der Fall, wenn in der Fig. 2 der Schleifstift 7 entlang seiner Rotationsachse nach unten verschoben wird. Man kann hier von einem vollständigen stirnseitigen Lastfall sprechen. In der anderen Situation nimmt nur ein Teil der Stirnfläche am Materialabtrag teil. Dies ist zum Beispiel dann der Fall, wenn der Schleifstift 7 in der Fig. 3 entlang seiner Rotationsrichtung nach unten verschoben wird. Hierbei erfolgt ein Abtrag in der Fig. 3 nur am rechten unteren Ende des Schleifstiftes.

Eine ähnliche Unterscheidung kann auch für das Verschwenken des Schleifstiftes in die Papierebene hinein oder aus ihr heraus getroffen werden. Wird zum Beispiel in der Fig. 2 der Schleifstift weit genug in die Papierebene hineinverschwenkt, so erfolgt ein Materialabtrag an einem verhältnismäßig großen Teil der Mantelfläche. Dieser Teil liegt in der Fig. 2 auf der rückwärtigen Seite des Schleifstiftes und ist durch die besondere Schraffur angedeutet. Es liegt also eine hohe mantelseitige Belastung vor. In der Fig. 3 erfolgt dagegen ein Materialabtrag nur mit einem verhältnismäßig kleinen Anteil der Mantelfläche des Schleifstiftes 7, der wiederum durch die Schraffur angedeutet ist.

Es ist also ersichtlich, daß im Verlaufe der Bearbeitung des Werkstückes sehr unterschiedliche Belastungen des Werkzeuges (Schleifstiftes 7) auftreten können.

Um zu verhindern, daß dabei eine Überbelastung des Werkzeuges auftritt, sind nun verschiedene Varianten denkbar. Die erste und einfachste Variante besteht darin, während der gesamten Bearbeitung die Geschwindigkeit der Relativbewegung zwischen Werkzeug und Werkstück auf einen geringen Maximalwert zu begrenzen. Bei genügend niedriger Wahl dieser Geschwindigkeit führt dies dazu, daß selbst im ungünstigsten Lastfall keine Überbelastung des Werkzeuges erfolgen kann. Durch ein solches Vorgehen kann man zwar eine Überlastung des Werkzeuges wirksam verhindern, jedoch erhöht sich die Bearbeitungszeit des Werkstückes in inakzeptabler Weise.

Eine zweite Variante, die gemäß dem Stand der Technik bislang praktiziert wurde, besteht darin, die Belastung des Werkzeugs kontinuierlich zu ermitteln und die Relativbewegung zwischen Werkzeug und Werkstück anhand der ermittelten Belastung zu regeln. Dazu wird beispielsweise die Rotationsfrequenz des Werkzeuges so geregelt, daß diese weitgehend konstant bleibt; es wird dann die Leistungsaufnahme des Werkzeugantriebs ermittelt, zum Beispiel durch Messung des Stromes bei einem elektrischen Werkzeugantrieb. Diese Leistungsaufnahme ist ein unmittelbares Maß für die momentane Belastung des Werkzeuges. Die Regelung der Relativgeschwindigkeit zwischen Werkzeug und Werkstück erfolgt dann so, daß bei einer hohen Belastung die Relativgeschwindigkeit zwischen Werkzeug und Werkstück erniedrigt wird und umgekehrt. Bei einem idealen, verzögerungsfreien Funktionieren einer solchen Regelung könnte das Werkstück mit einer konstanten Abtragsleistung und konstanten Belastung des Werkzeuges bearbeitet werden.

Insbesondere bei schnellen Lastwechseln reicht diese Regelung der Relativgeschwindigkeit alleine nicht aus. Sie wird gemäß der vorliegenden Erfindung um eine Vorausbestimmung der zu erwartenden Abtragsleistung bzw.

Werkzeugbelastung ergänzt. Auf diese Art und Weise kann die Bewegung zwischen Werkzeug und Werkstück in denjenigen Bereichen, in denen eine hohe Abtragsleistung zu erwarten ist, von vornherein sehr langsam angeführt werden und umgekehrt in Bereichen, in denen nur eine niedrige Abtragsleistung erwartet wird, eine höhere Relativgeschwindigkeit zwischen Werkzeug und Werkstück gewählt werden.

Der Ablauf des erfindungsgemäßen Verfahrens ist schematisch in der Fig. 4 illustriert. Die geometrischen Daten des zu erstellenden Paßkörpers sind in einer Rechen- und Speichereinheit 10 gespeichert. Im Falle der Fertigung eines zahnmedizinischen Prothetikpaßkörpers können diese Daten beispielsweise auf an sich bekannte Weise aus einer optischen 3D-Aufnahme eines Bereiches der Mundhöhle gewonnen werden. Aus diesen geometrischen Daten errechnet die Rechen- und Speichereinheit 10 einerseits die Bahn, auf der sich das Bearbeitungswerkzeug und das Werkzstück relativ zueinander bewegen, sowie andererseits für eine Anzahl von Punkten dieser Bahn die zu erwartende Abtragsleistung des Werkzeuges. Schließlich errechnet die Rechen- und Steuereinheit 10 hieraus Steuersignale, die sie an eine Regelungseinheit 11 weitergibt. Die Regelungseinheit 11 regelt verschiedene elektromotorische, hydraulische oder pneumatische Antriebe, die zur Ausführung einer Relativbewegung zwischen Werkzeug und Werkstück vorgesehen sind. In der Fig. 4 sind diese Antriebe schematisch zu einer Antriebseinheit 12 zusammengefaßt. Neben den Steuersignalen aus der Rechen- und Steuereinrichtung 10 erhält die Regelungseinrichtung 11 weiterhin auch Signale von einer Antriebsregelung 13, mit deren Hilfe der elektromotorische Rotationsantrieb 14 des Bearbeitungswerkzeuges geregelt wird. Die Regelung erfolgt in einer solchen Weise, daß die Rotationsfrequenz des Bearbeitungswerkzeuges weitgehend konstant bleibt. Der Regelungseinheit 11 werden dann Signale zugeführt, die Informationen über die Leistungsaufnahme des Rotationsantriebes 14 enthalten. Aus den Signalen der Rechen- und Steuereinheit 10 einerseits und der Antriebsregelung 13 andererseits ermittelt die Regelungseinheit 11 die notwendigen Stellgrößen für die Antriebseinheit 12, die erforderlich sind, um die Relativbewegung zwischen Werkzeug und Werkstück entlang der berechneten Bahn auszuführen.

Eine Methode zur Berechnung der zu erwartenden Abtragsleistung in der Rechen- und Steuereinrichtung 10 wird im folgenden näher erläutert.

Die Bahn wird in der Rechen- und Steuereinrichtung 10 zunächst in eine Vielzahl von Abschnitten aufgeteilt, während derer kein linearer Vorschub des Werkstücks entlang der Vorschubsrichtung erfolgt. Für jeden Abschnitt kann nun punktweise die Berechnung der Schwenkposition des Werkzeuges sowie die Berechnung der Tiefe des Werkzeuges im Werkstück erfolgen. Prinzipiell läßt sich die Bahn also darstellen durch eine Vielzahl von Zahlentripeln, wobei der erste Zahlenwert jeden Tripels den Bahnabschnitt, der zweite Zahlenwert die Schwenkposition des Werkzeuges und der dritte Zahlenwert die Tiefe des Werkzeugs im Werkstück angibt. Falls das Werkstück zusätzlich eine Drehung um die Vorschubachse ausführt, kommt hierzu ein weiterer Zahlenwert für den Drehwinkel des Werkstücks.

Die Berechnung der Bahndaten muß dabei nicht für die gesamte Bahn vollständig vor Beginn des Schleifvorgangs erfolgen. Sie kann auch abschnittsweise durchgeführt werden, beispielsweise während des Ausschleifens eines Bahnabschnittes jeweils für den nächsten Bahnabschnitt.

Zur Erläuterung der Regelung der Relativbewegung zwischen Werkzeug und Werkstück sei nun zunächst angenommen, daß die Bearbeitung einer bestimmten Anzahl von Bahnabschnitten schon erfolgt ist. In der Rechen- und Steuereinheit 10 sind zu diesem Zeitpunkt zumindest die Bahndaten des zuletzt ausgearbeiteten Bahnabschnittes sowie die Bahndaten des nächsten zu bearbeitenden Bahnabschnittes gespeichert. Die Rechen- und Steuereinheit 10 vergleicht nun punktweise für jede Schwenkposition des Schleifstiftes die Tiefe des Schleifstiftes im Werkstück auf der zuletzt ausgearbeiteten und auf der nächsten auszuarbeitenden Bahn. Durch diesen Vergleich kann zweierlei erkannt werden:
- Für jeden Punkt der Bahn läßt sich zunächst berechnen, ob ein Materialabtrag mit der Stirnseite des Schleifstiftes erfolgt. Falls dies der Fall ist, kann weiterhin näherungsweise derjenige Anteil der Stirnfläche des Schleifstiftes berechnet werden, an dem ein Materialabtrag stattfindet. In die Rechen- und Steuereinheit 10 wurde zuvor eine Tabelle eingespeichert, in der für verschiedene Anteile der Stirnfläche, an denen ein Abtrag stattfindet, die jeweils maximal zulässige Geschwindigkeit für die Tiefenänderung des Schleifstiftes im Werkstück eingetragen ist. Diese Maximalgeschwindigkeit wurde zuvor für das jeweilige Werkstückmaterial empirisch im Labor ermittelt.
- Ebenfalls ermöglicht der abschnittsweise Vergleich der Bahndaten die Berechnung desjenigen Anteils der Mantelfläche, an dem ein Materialabtrag stattfindet. In der Rechen- und Steuereinheit 10 wurde zuvor eine weitere Tabelle eingetragen, in der für verschiedene Werte des Anteils der Mantelfläche die jeweils maximale zulässige Geschwindigkeit für die Schwenkbewegung des Werkzeuges relativ zum Werkstück eingetragen ist. Durch Vergleich mit dieser Tabelle kann so für jeden Punkt der Bahn die maximal zulässige Schwenkgeschwindigkeit ermittelt werden.

Falls zusätzlich eine Drehung des Werkstücks um die Vorschubachse V ausgeführt wird, gilt das Gesagte analog auch für die maximale Geschwindigkeit für die Drehung des Werkstücks um diese Achse.

Die Rechen- und Steuereinrichtung 10 leitet nun Signale an die Regelungseinheit 11 weiter, die geeignet sind, den Antrieb für die Verschwenkung des Werkzeuges und die Tiefenänderung des Werkzeuges anzusteuern. Falls der Antrieb beispielsweise Schrittmotoren enthält, liefert die Rechen- und Steuereinrichtung 10 an die Regelungseinrichtung 11 Informationen über die an jedem Bahnpunkt vorzunehmende Anzahl von Verstellschritten des Schrittmotors und die zeitliche Abfolge dieser Verstellschritte. Die zeitliche Abfolge der Verstellschritte legt dabei insbesondere die Geschwindigkeit der Relativbewegung fest. Sie wird so gewählt, daß diese Geschwindigkeit nahe, aber unterhalb der berechneten maximal zulässigen Geschwindigkeit liegt.

Die Regelungseinrichtung 11 steuert mit Hilfe dieser Signale unmittelbar die Antriebe für die Verschwenkbewegung und die Tiefenänderung des Werkzeuges sowie nach Ende eines jeden Bahnabschnitts den Antrieb für den Werkstückvorschub an. Dabei erhält sie aus der Regelungseinrichtung 13 Informationen über die momentane Belastung des Schleifstiftes. Bei optimalen Bedingungen, das heißt bei exakter Justierung der Werkzeuge, einem neuen und damit leistungsstarken Schleifstift, optimaler Kühlung usw. wird die ermittelte momentane Belastung weitgehend mit der berechneten Belastung übereinstimmen. Die Regelungseinrichtung braucht in diesem Fall nicht einzugreifen. Ist jedoch der Schleifstift abgenutzt und daher weniger leistungsfähig als erwartet, oder liegen sonstige nicht optimale Betriebsbedingungen vor, so wird die tatsächliche Belastung des Schleifstiftes höher als die berechnete Belastung sein. In diesem Fall greift die Regelungseinrichtung 11 regelnd ein, in dem sie die Relativgeschwindigkeit zwischen Werkzeug und Werkstück absenkt.

Die Kombination aus einer Vorausberechnung der zu erwartenden Belastung und einer Ermittlung der tatsächlichen momentanen Belastung zum Zwecke der Regelung der Relativbewegung zwischen Werkzeug und Werkstück bringt eine Vielzahl von Vorteilen mit sich, von denen hier einige genannt seien:
- Die Bewegungsgeschwindigkeit des Schleifstifts kann an Positionen, an denen nur ein geringer Materialabtrag erfolgt, erheblich gesteigert werden. Hierdurch ergibt sich eine kürzere Gesamtbearbeitungsdauer bei der Fertigung eines Werkstücks. Dies ist insbesondere dann von Bedeutung, wenn aus dem Werkstück ein zahnärztlicher Prothetikpaßkörper gefertigt werden soll, der unmittelbar in der Praxis des Zahnarztes im Mund des Patienten eingesetzt werden soll.
- Die Kräfte zwischen Werkstück und Schleifstift werden insbesondere an solchen Positionen, an denen viel Material abgetragen werden muß, durch die dort reduzierte Bewegungsgeschwindigkeit erheblich vermindert. Dies ist insbesondere dann von Bedeutung, wenn filigrane Formen mit geringer Materialstärke ausgearbeitet werden sollen. Bei übermäßiger Krafteinwirkung zwischen Schleifstift und Werkstück können solche Formen leicht abbrechen. Ebenso wird ein Abbrechen des Schleifstiftes verhindert.
- In jeder denkbaren Betriebssituation ist eine ausreichende Kühlung des Schleifstiftes gewährleistet. Bei der Erkennung einer starken Belastung des Schleifstiftes kann die Regelungseinrichtung 11 so eingestellt werden, daß unter Umständen sogar ein vollständiges Zurückziehen des Schleifstiftes aus dem Werkzeug erfolgt. Dies erlaubt eine schnelle Kühlung der belasteten Flächen und das Ausspülen von abgetragenen Partikeln aus Vertiefungen im Werkstück.
- Das Verfahren ist nicht nur bei Einsatz eines Schleifstiftes anwendbar, sondern ebensogut auch für andere Werkzeuge wie Fräser oder Schleif- und Trennscheiben.
- Schließlich ermöglicht das vorgeschlagene Verfahren eine selbsttätige Erkennung der Abnutzung des Schleifstiftes. Eine solche Erkennung kann über die Überwachung der Häufigkeit des Eingreifens der Regelung 11 aufgrund einer übermäßigen Belastung des Schleifstiftes erfolgen. Bei einem zu häufigen Eingreifen der Regelung über einen längeren Zeitraum kann der Benutzer durch eine geeignete Signaleinrichtung dazu aufgefordert werden, den Schleifstift zu wechseln.

Das erfindungsgemäße Regelungsverfahren kann häufig ohne apparative Änderung an bestehenden Vorrichtungen zur Erstellung von medizinischen Paßkörpern implementiert werden. Eine solche Vorrichtung wird in jedem Falle ohnehin eine Rechen- und Steuereinrichtung umfassen, die nach geeigneter Programmierung die Funktionalität der Rechen- und Steuereinrichtung 10 der Fig. 4 übernehmen kann. Gleichfalls wird in vielen bestehenden Vorrichtungen schon eine Regelungseinheit zur Erkennung von Überlast vorhanden sein. Statt einer festen maximalen Relativgeschwindigkeit zwischen Werkstück und Werkzeug wird dieser Regelungseinheit nun eine variable maximale Relativgeschwindigkeit vorgegeben, die vom jeweiligen Bahnpunkt abhängig ist. So kann auf einfache Weise und mit geringem Kostenaufwand eine sehr zuverlässige Regelung erreicht werden.

### Bezugszeichenliste

- 1: Werkstück
- 2: Halter
- 3: zu erstellende Kontur
- 4 - 6: Bahnabschnitte
- 7: Schleifstift
- 8, 8': herausgearbeitete Kontur
- 9: Rotationsachse
- A, B, C: Bearbeitungspunkte
- V: Vorschubsrichtung

## Patentansprüche

1. Verfahren zur Erstellung von medizinischen, insbesondere zahnmedizinischen Paßkörpern, bei dem ein Werkstück (1), aus dem der Paßkörper erstellt wird, mittels eines Werkzeuges (7) abtragend bearbeitet wird, wobei das Werkzeug (7) und das Werkstück (1) eine Relativbewegung entlang einer Bahn ausführen und wobei die tatsächliche Belastung des Werkzeuges (7) zur Regelung der Geschwindigkeit der Relativbewegung herangezogen wird, **dadurch gekennzeichnet, dass** die zu erwartende Belastung des Werkzeuges (7) entlang der Bahn berechnet und zur Steuerung der Relativgeschwindigkeit herangezogen wird, wobei eine Regelungseinrichtung (11) vorgesehen ist, mittels derer die berechnete Belastung des Werkzeugs (7) mit der tatsächlichen Belastung verglichen wird.

2. Verfahren nach Anspruch 1, **dadurch gekennzeichnet, daß** die Relativbewegung zwischen dem Werkzeug (7) und dem Werkstück (1) folgende Bewegungsarten umfaßt:
a) linearer Vorschub zwischen Werkzeug (7) und Werkstück (1) entlang einer Vorschubrichtung (V), die im wesentlichen senkrecht zur Symmetrieachse (9) des Werkzeuges (7) ist;
b) Tiefenänderung des Werkzeuges (7) im Werkstück (1) im wesentlichen entlang der Symmetrieachse (9) des Werkzeuges (7);
c) eindimensionale Seitwärtsbewegung zwischen dem Werkzeug (7) und dem Werkstück (1) entlang eines vorgegebenen Weges (5; 5; 6) in einer Ebene, die im wesentlichen senkrecht zur Symmetrieachse (9) des Werkzeuges (1) ist.

3. Verfahren nach Anspruch 2, **dadurch gekennzeichnet, daß** die eindimensionale Seitwärtsbewegung in einer Verschwenkung des Werkzeuges (7) um eine Achse, die im wesentlichen parallel zur Symmetrieachse (9) des Werkzeuges (7) ist, besteht.

4. Verfahren nach Anspruch 2 oder 3, **dadurch gekennzeichnet, daß** die Relativbewegung zwischen dem Werkzeug (7) und dem Werkstück (1) weiterhin eine Drehung des Werkstückes (1) um eine Achse umfaßt, die im wesentlichen parallel zur Vorschubrichtung (V) ist.

5. Verfahren nach einem der Ansprüche 2 bis 4, **dadurch gekennzeichnet, daß** das Werkzeug (7) ein im wesentlichen zylindrischer Schleifstift mit einer Mantelfläche und einer Stirnfläche ist.

6. Verfahren nach Anspruch 5, **dadurch gekennzeichnet,**
- **daß** der lineare Vorschub in Schritten erfolgt, die klein gegenüber dem Durchmesser des Schleifstiftes sind und die von der gewünschten Präzision bei der Herausarbeitung der Kontur abhängen,
- **daß** die Bahn in Abschnitte unterteilt ist, zwischen denen kein linearer Vorschub erfolgt
- und **daß** die zu erwartende Belastung für jeden Abschnitt aus dem Vergleich der Bahn entlang dieses Abschnitts und der Bahn entlang des vorhergehenden Abschnitts berechnet wird.

7. Verfahren nach Anspruch 6, **dadurch gekennzeichnet, daß** zur Bestimmung der zu erwartenden Belastung bei einer eindimensionalen Seitwärtsbewegung die Höhe desjenigen Anteils der Mantelfläche des Schleifstiftes berechnet wird, an dem ein Materialabtrag erfolgt.

8. Verfahren nach Anspruch 7, **dadurch gekennzeichnet, daß** die Geschwindigkeit der eindimensionalen Seitwärtsbewegung auf eine Maximalgeschwindigkeit begrenzt wird, die unter Zugriff auf eine Tabelle ermittelt wird, in der für mehrere Werte der Höhe desjenigen Anteils der Mantelfläche, an dem ein Materialabtrag erfolgt, die zugeordnete Maximalgeschwindigkeit gespeichert ist.

9. Verfahren nach einem der Ansprüche 6 bis 8, **dadurch gekennzeichnet, daß** zur Bestimmung der zu erwartenden Belastung bei einer Tiefenänderung des Schleifstiftes im Werkstück (1) näherungsweise derjenige Anteil der Stirnfläche bestimmt wird, an dem ein Materialabtrag erfolgt.

10. Verfahren nach Anspruch 9, **dadurch gekennzeichnet, daß** die Geschwindigkeit der Tiefenänderung des Werkzeuges (7) im Werkstück (1) auf eine Maximalgeschwindigkeit begrenzt wird, die unter Zugriff auf eine Tabelle ermittelt wird, in der für mehrere Werte des Anteiles der Stirnfläche, an der ein Materialabtrag erfolgt, die zugeordnete Maximalgeschwindigkeit gespeichert ist.

11. Verfahren nach einem der Ansprüche 1 bis 10, **dadurch gekennzeichnet, dass** bei Erkennen einer starken Belastung mittels der Regelungseinrichtung (13) ein vollständiges Zurückziehen des Werkzeugs (7) aus dem Werkstück erfolgt.

12. Verfahren nach einem der Ansprüche 1 bis 11, **dadurch gekennzeichnet, dass** ein zu häufiges Eingreifen der Regelungseinrichtung (13) mittels einer Signaleinrichtung angezeigt wird.

## Claims

1. Method for the production of medical fittings, in particular dental fittings, in which a workpiece (1), from which the fitting is produced, has material removed from it by machining with a tool (7), the tool (7) and the workpiece (1) executing a relative movement along a trajectory, and the actual loading of the tool (7) being used to control the speed of the relative movement, **characterized in that** the expected loading of the tool (7) along the trajectory is calculated and is used to control the relative speed, a control device (11) being provided by means of which the calculated loading of the tool (7) is compared with the actual loading.

2. Method according to Claim 1, **characterized in that** the relative movement between the tool (7) and the workpiece (1) includes the following types of movement:
a) linear feed between the tool (7) and the workpiece (1) along a feed direction (V) which is essentially perpendicular to the axis of symmetry (9) of the tool;
b) change of depth of the tool (7) in the workpiece (1) essentially along the axis of symmetry (9) of the tool (7);
c) one-dimensional sideways movement between the tool (7) and the workpiece (1) along a predetermined path (5; 5; 6) in a plane which is essentially perpendicular to the axis of symmetry (9) of the tool (1).

3. Method according to Claim 2, **characterized in that** the one-dimensional sideways movement consists of a swivelling of the tool (7) about an axis which is essentially parallel to the axis of symmetry (9) of the tool (7).

4. Method according to Claim 2 or 3, **characterized in that** the relative movement between the tool (7) and the workpiece (1) moreover includes a pivoting of the workpiece (1) about an axis which is essentially parallel to the feed direction (V).

5. Method according to one of Claims 2 through 4, **characterized in that** the tool (7) is an essentially cylindrical grinder rod with a circumferential surface and an end surface.

6. Method according to Claim 5, **characterized in that**
- the linear feed takes place in steps which are small compared to the diameter of the grinder rod and which depend on the desired precision on production of the contour,
- the trajectory is divided into sections between which no linear feed takes place,
- and the expected loading for each section is calculated by comparison of the trajectory along this section and the trajectory along the previous section.

7. Method according to Claim 6, **characterized in that**, in order to determine the expected loading upon a one-dimensional sideways movement, the height of that portion of the circumferential surface of the grinder rod from which material is removed is calculated.

8. Method according to Claim 7, **characterized in that** the speed of the one-dimensional sideways movement is limited to a maximum speed which is determined by reference to a table in which, for a number of values of the height of that portion of the circumferential surface from which material is removed, the associated maximum speed is stored.

9. Method according to one of Claims 6 through 8, **characterized in that**, in order to determine the expected loading upon a change of depth of the grinder rod in the workpiece (1), that portion of the end face approximately is determined from which material is removed.

10. Method according to Claim 9, **characterized in that** the speed of the change of depth of the tool (7) in the workpiece (1) is limited to a maximum speed which is determined by reference to a table in which, for several values of that portion of the end face from which material is removed, the associated maximum speed is stored.

11. Method according to one of Claims 1 to 10, **characterized in that**, upon detection of a high loading of the tool, the control device (11) effects a complete withdrawal of the tool from the workpiece.

12. Method according to one of Claims 1 to 11, **characterized in that**, if the intervention of the control device (11) is too frequent, this is displayed by means of a signal device.

## Revendications

1. Procédé pour fabriquer des prothèses médicales, en particulier dentaires, dans lequel une pièce (1), à partir de laquelle la prothèse est fabriquée, est usinée par enlèvement de copeaux au moyen d'un outil (7), l'outil (7) et la pièce (1) effectuant un mouvement relatif le long d'une trajectoire et la sollicitation effective de l'outil (7) étant utilisée pour la régulation de la vitesse du mouvement relatif, **caractérisé en ce que** la sollicitation attendue de l'outil (7) est calculée le long de la trajectoire et est utilisée pour commander la vitesse relative, un dispositif de régulation (11) étant prévu, au moyen duquel la sollicitation calculée de l'outil (7) est comparée à la sollicitation effective.

2. Procédé selon la revendication 1, **caractérisé en ce que** le mouvement relatif entre l'outil (7) et la pièce (1) comprend les types de mouvement suivants :
a) avance linéaire entre l'outil (7) et la pièce (1) le long d'une direction d'avance (V) qui est essentiellement perpendiculaire à l'axe de symétrie (9) de l'outil ;
b) modification de la profondeur de l'outil (7) dans la pièce (1) essentiellement le long de l'axe de symétrie (9) de l'outil (7) ;
c) déplacement latéral unidimensionnel entre l'outil (7) et la pièce (1) le long d'un chemin prédéfini (5 ; 5 ; 6) dans un plan qui est essentiellement perpendiculaire à l'axe de symétrie (9) de l'outil (7).

3. Procédé selon la revendication 2, **caractérisé en ce que** le déplacement latéral unidimensionnel s'effectue suivant un pivotement de l'outil (7) autour d'un axe qui est essentiellement parallèle à l'axe de symétrie (9) de l'outil (7).

4. Procédé selon la revendication 2 ou 3, **caractérisé en ce que** le mouvement relatif entre l'outil (7) et la pièce (1) comprend en outre une rotation de la pièce (1) autour d'un axe qui est essentiellement parallèle à la direction d'avance (V).

5. Procédé selon l'une quelconque des revendications 2 à 4, **caractérisé en ce que** l'outil (7) est une broche de meulage essentiellement cylindrique avec une surface d'enveloppe et une face de bout.

6. Procédé selon la revendication 5, **caractérisé en ce que**
- l'avance linéaire s'effectue par pas qui sont petits par rapport au diamètre de la broche de meulage et qui dépendent de la précision souhaitée pour l'usinage du contour,
- la trajectoire est divisée en sections entre lesquelles ne se produit aucune avance linéaire,
- et la sollicitation attendue pour chaque section est calculée à partir de la comparaison de la trajectoire le long de cette section et de la trajectoire le long de la section précédente.

7. Procédé selon la revendication 6, **caractérisé en ce que** pour déterminer la sollicitation attendue dans le cas d'un déplacement latéral unidimensionnel, on calcule la hauteur de la proportion de la surface d'enveloppe de la broche de meulage sur laquelle s'effectue l'enlèvement de matière.

8. Procédé selon la revendication 7, **caractérisé en ce que** la vitesse du déplacement latéral unidimensionnel est limitée à une vitesse maximale qui est déterminée en se référant à une table dans laquelle est stockée la vitesse maximale associée pour plusieurs valeurs de la hauteur de la proportion de la surface d'enveloppe sur laquelle s'effectue un enlèvement de matière.

9. Procédé selon l'une quelconque des revendications 6 à 8, **caractérisé en ce que** pour la détermination de la sollicitation attendue dans le cas d'une modification de la profondeur de la broche de meulage dans la pièce (1), on détermine par approximation la proportion de la face de bout sur laquelle s'effectue un enlèvement de matière.

10. Procédé selon la revendication 9, **caractérisé en ce que** la vitesse de la modification de profondeur de l'outil (7) dans la pièce (1) est limitée à une vitesse maximale qui est déterminée en se référant à une table dans laquelle est stockée la vitesse maximale associée pour plusieurs valeurs de la proportion de la face de bout sur laquelle s'effectue un enlèvement de matière.

11. Procédé selon l'une quelconque des revendications 1 à 10, **caractérisé en ce que** dans le cas d'une détection d'une forte sollicitation de l'outil au moyen du dispositif de régulation (11), un retrait complet de l'outil hors de la pièce est effectué.

12. Procédé selon l'une quelconque des revendications 1 à 11, **caractérisé en ce qu'**une intervention trop fréquente du dispositif de régulation (11) est indiquée au moyen d'un dispositif de signalisation.
